(19) Europäisches Patentamt
European Patent Office
Office européen des brevets

(11) Veröffentlichungsnummer: **0 211 398**
**A2**

# (12) EUROPÄISCHE PATENTANMELDUNG

(21) Anmeldenummer: 86110547.6

(22) Anmeldetag: 30.07.86

(51) Int. Cl.⁴: C07F 9/17 , C07C 103/48 , C07D 333/24 , C07C 149/247 , A01N 57/12 , A01N 43/10 , A01N 37/46

(30) Priorität: 09.08.85 DE 3528631

(43) Veröffentlichungstag der Anmeldung: 25.02.87 Patentblatt 87/09

(84) Benannte Vertragsstaaten: BE CH DE FR GB IT LI

(71) Anmelder: **BASF Aktiengesellschaft**
**Carl-Bosch-Strasse 38**
**D-6700 Ludwigshafen(DE)**

(72) Erfinder: **Schulz, Guenter, Dr.**
**Carl-Bosch-Strasse 96**
**D-6700 Ludwigshafen(DE)**
Erfinder: **Fritsch, Hansjoerg, Dr.**
**Muehlweg 16**
**D-6703 Limburgerhof(DE)**
Erfinder: **Jung, Johann, Prof. Dr.**
**Hardenburgstrasse 19**
**D-6703 Limburgerhof(DE)**

## (54) Substituierte N-Acyl-aplha-aminosäureester, ihre Verwendung als Bioregulatoren insbesondere zur Senkung des endogenen Ethylenspiegels in Pflanzen.

(57) Substituierte N-Acyl--aminosäureester der Formeln Ia bzw. Ib

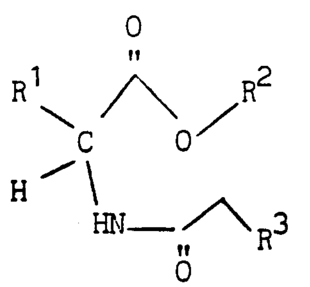

(Ia),

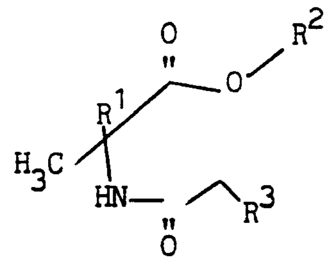

(Ib),

in denen

$R^1$ für Wasserstoff, n-$C_1$-$C_5$-Alkyl, Phenyl, Thiophen-3-yl, 2-Methylthio-ethyl-oder N-Chloracetyl-4-aminobutyl-,

$R^2$ für Methyl, Propyl, n-Butyl oder für $-(CH_2CH_2O)_n-R^4$, (n = 1 oder 2) steht, wobei

$R^4$ $C_1$ bis $C_4$-Alkyl, Phenyl, Methylphenyl oder Chlorphenyl bedeutet,

$R^3$ für Cl (außer wenn in Ia, $R^1$ = Phenyl, $R^2$ = $CH_2CH_2OR^4$ ist), Br, Methoxy oder für die Gruppe

EP 0 211 398 A2

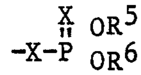

steht, wobei

$R^5$ und $R^6$ unabhängig voneinander $C_1$ -$C_6$ Alkyl bedeuten, sowie

X für Sauerstoff oder Schwefel steht,

insbesondere als Mittel zur Senkung des endogenen Ethylenspiegels in Pflanzen, ein Verfahren zur Herstellung der Verbindungen Ia bzw. Ib sowie die Verwendung der Verbindungen Ia bzw. Ib zur Beeinflussung des Pflanzenwachstums durch Einwirkung auf Pflanzen oder deren Saatgut.

# Substituierte N-Acyl-α-aminosäureester, ihre Verwendung als Bioregulatoren insbesondere zur Senkung des endogenen Ethylenspiegels in Pflanzen

Die Erfindung betrifft neue substituierte N-Acylaminosäureester und ihre Verwendung als Bioregulatoren, insbesondere zur Senkung des endogenen Ethylenspiegels in Pflanzen.

In grünen Pflanzen natürlicherweise gebildetes oder exogen zugeführtes Ethylen wirkt als Pflanzenhormon, das Seneszenz(Alterungs-)vorgänge in Pflanzen steuert. Die Senkung des endogenen Ethylenspiegels führt in der Regel zur Verzögerung des Seneszenz. Die Ausnutzung dieses Effektes kann auf verschiedene Weise erfolgen: als Beispiele seien die Verlängerung der Lebensdauer von Schnittblumen, die Verminderung der Fruchtabszission und die Verlängerung der reproduktiven Phase von Pflanzen zur Ernteertragssteigerung genannt. Bisher gelang die Senkung des endogenen Ethylenspiegels praktisch nur im Versuchsmaßstab; der technischen Anwendung der bekannten Wirkstoffe standen verschiedene Hindernisse im Wege: zum Beispiel die aufwendige Herstellung und die Humantoxizität im Falle des Aminoethoxyvinylglycins, die Phytotoxiztät und unspezifische Wirkung im Falle der Aminooxyessigsäure oder die Umweltbelastung im Falle von $Co^{++}$-Ionen [Literatur: Ann. Rev. Plant Physiol. 1984, (35), S. 155 bis 189]. Die aus den US-PSen 4 361 439, 4 385 922 und 4 396 414 bekannten Wirkstoffe beeinflussen nur wenige Pflanzenarten.

Es wurde nun gefunden, daß Wirkstoffe, die man als substituierte N-Acyl-α-aminosäureester bezeichnen kann und die die allgemeine Formel Ia bzw. Ib haben,

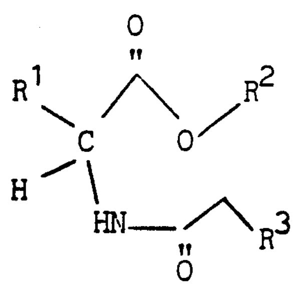

(Ia),

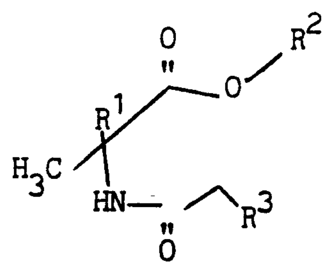

(Ib),

in denen

$R^1$ für Wasserstoff, n-$C_1$-$C_5$-Alkyl, Phenyl, Thiophen-3-yl, 2-Methylthio-ethyl oder N-Chloroacetyl-4-aminobutyl,

$R^2$ für Methyl, Propyl, n-Butyl oder für $-(CH_2CH_2O)_n-R^4$, (n = 1 oder 2) steht, wobei

$R^4$ $C_1$ bis $C_4$-Alkyl, Phenyl, Methylphenyl oder Chlorphenyl bedeutet,

$R^3$ für Cl (außer wenn in Ia $R^1$ = Phenyl, $R^2$ = $CH_2CH_2OR^4$ ist), Br, Methoxy oder für die Gruppe

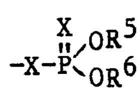

steht, wobei

$R^5$ und $R^6$ unabhängig voneinander $C_1$ -$C_6$ Alkyl bedeuten, sowie

X für Sauerstoff oder Schwefel steht.

bioregulierend im obengenannten Sinne wirken, sich daneben durch eine sehr gute Pflanzenverträglichkeit auszeichnen und auf einfache Art und Weise hergestellt werden können.

Eines von mehreren infragekommenden Herstellungsverfahren ist das Folgende:

Man geht aus von der entsprechenden Aminosäure, verestert diese in üblicher Weise unter Einführung des Restes $R^2$ und setzt den Aminosäureester mit einem entsprechend ($R^3$) substituierten Acylhalogenid um. Als Halogenid wird aus wirtschaftlichen Gründen i.a. das Chlorid, evtl. das Bromid verwendet. Gegebenenfalls setzt man weiter mit einem infragekommenden Phosphorsäurederivat um. Die benötigten Vorprodukte also Aminosäuren ggf. deren Ester einerseits und die Acylhalogenide, sowie die Phosphorsäurederivate sind meist handelsübliche Stoffe, jedenfalls aber beschrieben und mindestens mit den üblichen Mitteln des Fachmannes leicht erhältlich.

Die zunächst erhaltenen Wirkstoffe können in einigen Fällen, wie dies an den nachfolgenden Beispielen 28 und 29 gezeigt ist, durch weitere Umsetzung zu neuen gleichartigwirkenden Stoffen führen.

Je nach der Art der Substituenten R und R' treten die Vorprodukte und/oder die erfindungsgemäßen Wirkstoffe als Racemate oder in Form des optischen Antipoden auf, die i.a. unterschiedlich bzw. unterschiedlich stark wirksam sind.

Herstellungsbeispiel (in der nachstehenden Tabelle 1 als Nr. 28 aufgeführt)

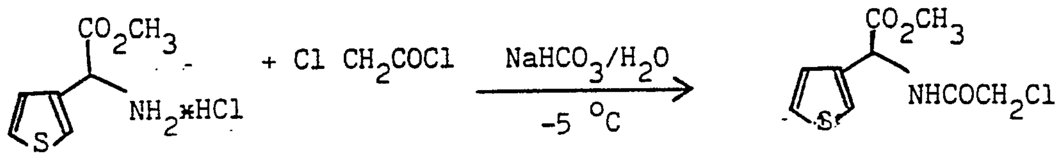


I II

Man löst 120 g (0.578 mol) I unter Zusatz von 97.1 g (0.156 mol) $NaHCO_3$ in 600 ml Wasser. Bei -5 °C wird langsam Chloracetylchlorid II (46.7 ml ≙ 0.578 mol) zugetropft. Man läßt Aufwärmen und rührt 1 bis 2 h nach. Der abgeschiedene Feststoff wird abgesaugt, mit Wasser und mit Pentan gewaschen und im Vakuum getrocknet: 107 g Nr. 28, Fp. 182 -185 °C.

Herstellungsbeispiel (in der Tabelle 1 als Nr. 29)

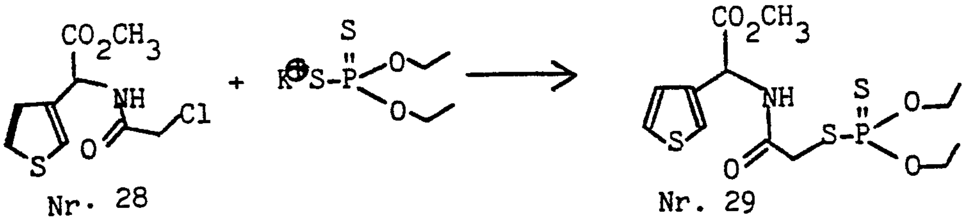

Nr. 28 Nr. 29

13.2 g (0.065 mol) Dithiophosphorsäure-diethylester werden mit 13.9 g (0.1 mol) Kaliumcarbonat in 200 ml Aceton ca. 1 h unter Rückfluß gerührt. Man setzt dann 10.8 g (0.05 mol) der Verbindung Nr. 28 zu und rührt 3 h bei Rückflußtemperatur. Nach Eindampfen wird in $CH_2Cl_2$ aufgenommen und zweimal mit $NaHCO_3$ -(wäßrig, 5prozentig) ausgeschüttelt. Trocknen der organischen Phase mit $MgSO_4$ und Eindampfen liefert 13 g Nr. 29 als wachsartigen Feststoff.

Nach den vorstehenden Angaben wurden durch entsprechende Abwandlung die in der nachstehenden Tabelle 1 aufgeführten weiteren Stoffe hergestellt.

Tabelle 1

| Nr. | R | $R^1$ | $R^2$ | $R^3$ | Fp. [°C] o. Aggregatzustand |
|---|---|---|---|---|---|
| 1 | -H | -H | $-CH_2CH_2OCH_3$ | $-SP(S)(OC_2H_5)_2$ | Öl |
| 2 | -H | -H | $-CH_2CH_2OCH_3$ | $-SP(S)(OCH(CH_3)_2)_2$ | Öl |
| 3 | -H | -H | $-CH_2CH_2OCH_3$ | $-SP(S)(OCH_2CH(CH_3)_2)_2$ | Öl |
| 4 | -H | -H | $-CH_2CH_2OCH_2CH_2OCH_3$ | $-SP(S)(OC_2H_5)_2$ | Öl |
| 5 | -H | -H | $-CH_2CH_2OCH_2CH_2OCH_3$ | $-SP(S)(OCH(CH_3)_2)_2$ | Öl |
| 6 | -H | -H | $-CH_2CH_2OCH_2CH_2OCH_3$ | $-SP(S)(OCH_2CH(CH_3)_2$ | Öl |
| 7 | -H | -H | $-CH_2CH_2-O-C_6H_5$ | $-SP(S)(OC_2H_5)_2$ | Öl |
| 8 | -H | -H | $-CH_2CH_2-O-C_6H_5$ | $-SP(S)(OCH(CH_3)_2)_2$ | Öl |
| 9 | -H | -H | $-CH_2CH_2-O-C_6H_5$ | $-SP(S)(OCH_2CH(CH_3)_2)_2$ | Öl |
| 10 | -H | -H | $-CH_2CH_2OCH_3$ | -Cl | Öl |
| 11 | -H | -H | $-CH_2CH_2OCH_2CH_2OCH_3$ | -Cl | Öl |
| 12 | -H | -H | $-CH_2CH_2O-C_6H_5$ | -Cl | Öl |
| 13 | -H | -H | $-CH_2CH_2O-(2-CH_3)C_6H_4$ | -Cl | 175 |
| 14 | -H | -H | $-CH_2CH_2CH_3$ | -Cl | wachsartig |
| 15 | H | $-C_6H_5$ | $-CH_2CH_2CH_3$ | $-SP(S)(OC_2H_5)_2$ | Öl |
| 16 | H | $-C_6H_5$ | $-CH_2CH_2OCH_2CH_2OCH_3$ | $-SP(S)(OC_2H_5)_2$ | Öl |
| 17 | H | $-C_6H_5$ | $-CH_2CH_2OCH_2CH_2OCH_3$ | $-SP(S)(OCH(CH_3)_2)_2$ | Öl |

| Nr. | R | $R^1$ | $R^2$ | $R^3$ | Fp. [°C] o. Aggregatzustand |
|---|---|---|---|---|---|
| 18 | H | $-C_6H_5$ | $-CH_2CH_2OCH_2CH_2OCH_3$ | $-SP(S)(OCH_2CH(CH_3)_2)_2$ | öl |
| 19 | H | $-C_6H_5$ | $-CH_2CH_2-O-C_6H_5$ | $-SP(S)(OC_2H_5)_2$ | öl |
| 20 | H | $-C_6H_5$ | $-CH_2CH_2-O-C_6H_5$ | $-SP(S)(OCH(CH_3)_2)_2$ | öl |
| 21 | H | $-C_6H_5$ | $-CH_2CH_2-O-C_6H_5$ | $-SP(S)(OCH_2CH(CH_3)_2)_2$ | öl |
| 22 | H | $-C_6H_5$ | $-CH_3$ | $-SP(S)(OC_2H_5)_2$ | wachsartig |
| 23 | H | $-C_6H_5$ | $-CH_3$ | $-Cl$ | 111 |
| 24 | H | $-C_6H_5$ | $-CH_2CH_2CH_3$ | $-Cl$ | 57 |
| 25 | H | $-C_6H_5$ | $-CH_2CH_2OCH_2CH_2OCH_3$ | $-Cl$ | öl |
| 26 | H | $-C_6H_5$ | $-CH_2CH_2OC_6H_5$ | $-Cl$ | 103 - 106 |
| 27 | H | $-C_6H_5$ | $-CH_2CH_2OCH_3$ | $-SP(S)(OC_2H_3)_2$ | öl |
| 28 | H | Thiophen-3-yl | $-CH_3$ | $-Cl$ | 182 - 185 |
| 29 | H | Thiophen-3-yl | $-CH_3$ | $-SP(S)(OC_2H_5)_2$ | wachsartig |
| 30 | H | Thiophen-3-yl | $-CH_3$ | $-SP(S)(OCH(CH_3)_2)_2$ | wachsartig |
| 31 | H | Thiophen-3-yl | $-CH_3$ | $-SP(S)(OCH_2CH(CH_3)_2)_2$ | wachsartig |
| 32 | H | $-CH_2CH_2CH_2CH_3$ | $-CH_3$ | $-Cl$ | 63° |
| 33 | H | $-CH_2CH_2SCH_3$ | $-CH_3$ | $-Cl$ | öl |
| 34 | H | $CH_2CH_2CH_2CH_2N(H)COCH_2Cl$ | $-CH_3$ | $-Cl$ | öl |
| 35 | $-CH_3$ | $-CH_3$ | $-CH_3$ | $-Cl$ | öl |

Die in den Beispielen der vorstehenden Tabelle 1 als Öle oder Wachse bezeichneten Stoffe sind durch Aufnahme eines $^1$H-NMR-Spektrums in $CDCl_3$ charakterisiert worden; die Angaben sind in Tabelle 2 zusammengefaßt:

Tabelle 2

| Nr. | $^1$H-NMR-Spektrum in $CDCl_3$ |
|---|---|
| 1 | 1.40 t (6H), 3.40 s (3H), 7.2-7.5 m (1H) |
| 2 | 1.35 d (12H), 3.35 s (3H), 4.80 hept. (2H), 7.0-7.25 m (1H) verbr. |
| 3 | 0.98 d (12H), 2.00 m(2H), 3.45 s (3H), 6.9-7.1 m (1H) verbr. |
| 4 | 1.38 d/1.40 d (6H), 3.4 s (3H), 7.1 t (1H) verbr. |
| 5 | 1.35 d (12H), 3.35 s (3H), 4.83 hept. (2H), 7.15 m (1H) verbr. |
| 6 | 0.9 d (12H), 2.0 m (2H), 3.35 s (3H), 7.25 t/7.30 t (1H) verbr. |
| 7 | 1.34 d/1.37 d (12H), 4.5 hept. (2H), 6.8-7.4 m (5H), 7.0 t (1H) verbr. |
| 8 | 1.35 d (12H), 4.8 hept. (2H), 6.8-7.4 m (5H), 6.9 t (1H) verbr. |
| 9 | 0.95 d (12H), 2.0 m (2H), 6.8-7.4 m (5H), 7.0 t (1H) verbr. |
| 10 | 3.38 s (3H), 4.10 s (2H), 7.0-7.4 m (1H) verbr. |
| 11 | 3.4 s (3H), 4.12 s (2H), 7.4-7.8 m (1H) verbr. |
| 12 | 4.15 s (2H), 6.8-7.45 m (5H), 8.7 t (1H) verbr. |
| 14 | 1.0 t (3H), 4.1 s (2H), 7.3-7.7 m (1H) verbr. |
| 15 | 0.85 t (3H), 1.25 t/1.30 t (6H), 4.05 s (2H), 5.48 d (1H), 7.28 s (5H) |
| 16 | 1.20 t/1.25 t (6H), 3.25 s (3H), 5.45 d (1H), 7.25 s (5H) |
| 17 | 1.2 m (12H), 3.35 s (3H), 4.7 m (2H), 5.45 d (1H), 7.20 s (5H) |
| 18 | 0.98 d (12H), 1.98 m (2H), 3.35 s (3H), 5.55 d (1H), 7.33 s (5H) |
| 19 | 1.20 t/1.22 t (6H), 5.5 d (1H), 6.6-7.5 m (11H) |
| 22 | 1.28 t/1.30 t (6H), 3.72 s (3H), 5.55 d (1H), 7.48 s (5H) |
| 25 | 3.43 s (3H), 4.20 s (2H), 5.52 d (1H), 7.4 s (5H), 9.0 d (1H) verbr. |
| 27 | 1.20 t/1.25 t (6H), 3.15 s (3H), 5.45 d (1H), 7.23 s (5H) |
| 29 | 1.23 t/1.28 t (6H), 3.70 s (3H), 5.65 d (1H), 7.0-7.4 m (3H), 7.6 d (1H) verbr. |
| 30 | 1.25 d/1.30 d (12H), 4.7 m (2H), 3.65 s (3H), 5.65 d (1H), 6.9-7.3 m (3H), 7.55 d (1H) verbr. |
| 31 | 0.88 d (12H), 1.90 m (2H), 3.65 s (3H), 5.60 d (1H), 6.9-7.3 m (3H), 7.45 d (1H) verbr. |
| 33 | 2.15 s (3H), 3.85 s (3H), 4.10 s (2H), 4.80 m (1H), 7.35 m (1H) verbr. |
| 34 | 3.75 s (3H), 4.10 s (4H), 7.4-8.0 (2H) breit |
| 35 | 1.65 s (6H), 3.75 s (3H), 4.03 s (2H) |

Die Hemmwirkung der Stoffe nach Anspruch 1 auf die Ethylenbiosynthese kann durch einen Test an abgeschnittenen Sojablättern quantifiziert werden. Man verfährt dazu wie folgt: Sojablattstücke werden 18 h in Gegenwart von Wirkstoff inkubiert und dann in ein geschlossenes Gefäß überführt. Nach weiteren 4 h mißt man die Ethylenkonzentration im Gasraum des Gefäßes mittels quantitativer Gaschromatographie. Einige Ergebnisse sind als Beispiele in der anschließenden Tabelle aufgeführt.

| Verbindung | % Hemmung |
|---|---|
| Wasser | 0 |
| Aminooxyessigsäure | 85 |
| 6 | 94 |
| 10 | 91 |
| 11 | 89 |
| 14 | 95 |
| 32 | 95 |
| 34 | 85 |

Wie die Beispiele zeigen, führen die Wirkstoffe zu einer drastischen Senkung der Ethylenakkumulation. Die Wirkung der Vergleichssubstanz wird erreicht oder übertroffen. Dabei ist Pflanzenverträglichkeit der neuen Wirkstoffe viel besser.

Damit ist der Einsatz z.B. in Getreide wie Weizen, Gerste, Roggen, Hafer, Mais, Reis möglich, wo bekanntermaßen durch Temperaturstreß via Streßethylen eine verfrühte Abreife induziert wird. Die Unterdrückung der Streßethylenbildung kann zur Verhinderung von Ertragseinbußen genutzt werden.

**Ansprüche**

1. Substituierte N-Acyl-α-aminosäureester der Formeln Ia bzw. Ib

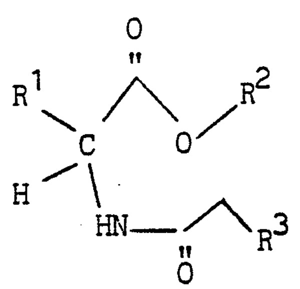

(Ia),

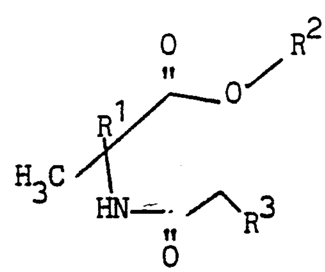

(Ib),

in denen

$R^1$ für Wasserstoff, n-$C_1$-$C_5$-Alkyl, Phenyl, Thiophen-3-yl, 2-Methylthio-ethyl-oder N-Chloracetyl-4-aminobutyl-,

$R^2$ für Methyl, Propyl, n-Butyl oder für $-(CH_2CH_2O)_n$-$R^4$, (n = 1 oder 2) steht, wobei

$R^4$ $C_1$ bis $C_4$-Alkyl, Phenyl, Methylphenyl oder Chlorphenyl bedeutet,

$R^3$ für Cl (außer wenn in Ia, $R^1$ = Phenyl, $R^2$ = $CH_2CH_2OR^4$ ist), Br, Methoxy oder für die Gruppe

steht, wobei

$R^5$ und $R^6$ unabhängig voneinander $C_1$ -$C_6$ Alkyl bedeuten, sowie

X für Sauerstoff oder Schwefel steht.

2. Mittel, insbesondere zur Senkung des endogenen Ethylenspiegels in Pflanzen enthaltend mindestens eine Verbindung der Formel 1a oder 1b gemäß Anspruch 1.

3. Verfahren zur Herstellung der Verbindungen des Anspruches 1, dadurch gekennzeichnet, daß man in an sich bekannter Weise eine entsprechende Aminosäure mit einem entsprechendem Alkohol $R^2OH$ verestert und mit einem entsprechendem Acylhalogenid acyliert und gegebenenfalls mit einem entsprechenden Phosphorsäurederivat umsetzt.

4. Verfahren zur Beeinflussung des Pflanzenwachstums, dadurch gekennzeichnet, daß man mindestens eine Verbindung gemäß Anspruch 1 bzw. ein Mittel gemäß Anspruch 2 auf Pflanzen oder deren Saatgut einwirken läßt.

5. Verfahren zur Herstellung eines Mittels gemäß Anspruch 2, dadurch gekennzeichnet , daß man ein N-Acyl--aminosäurederivat gemäß Anspruch 1 mit festen oder flüssigen Trägerstoffen sowie gegebenenfalls mit einem oder mehreren oberflächenaktiven Mitteln mischt.